(19) Europäisches Patentamt
European Patent Office
Office européen des brevets

(11) Veröffentlichungsnummer: **0 173 324**
**A1**

(12) # EUROPÄISCHE PATENTANMELDUNG

(21) Anmeldenummer: **85110838.1**

(22) Anmeldetag: **19.08.85**

(51) Int. Cl.⁴: **C 07 D 239/42**, C 07 D 239/47, C 07 D 239/48, C 07 D 239/54, C 07 D 251/16, C 07 D 251/18, C 07 D 251/22, C 07 D 251/46, C 07 D 251/50, C 07 D 213/75, A 01 N 47/36

(30) Priorität: **30.08.84 DE 3431932**

(43) Veröffentlichungstag der Anmeldung: **05.03.86 Patentblatt 86/10**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Hoehe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**

(54) **Verfahren zur Herstellung von 1-(2-Oxyaminosulfonyl-phenyl-sulfonyl)-3-heteroaryl-harnstoffen.**

(57) Die Erfindung betrifft ein neues Verfahren zur Herstellung von 1-(2-Oxyaminosulfonyl-phenylsulfonyl)- 3-heteroaryl-harnstoffen der allgemeinen Formel (I)

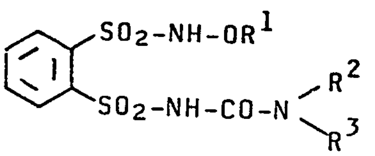

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für einen gegebenenfalls substituierten und/oder gegebenenfalls anellierten sechsgliedrigen aromatischen Heterocyclus, welcher wenigstens ein Stickstoffatom enthält, steht, dadurch gekennzeichnet, dass man Benzodisultame der Formel (II)

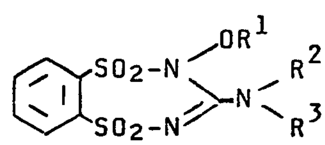

(II)

in welcher

$R^1$, $R^2$ und $R^3$ die oben angegebenen Bedeutungen haben, mit Wasser gegebenenfalls in Gegenwart von Basen und gegebenenfalls in Gegenwart von Verdünnungsmitteln bei Temperaturen zwischen 0 °C und 100 °C umsetzt.

EP 0 173 324 A1

0 173 324

BAYER AKTIENGESELLSCHAFT
Konzernverwaltung RP
Patentabteilung

5090 Leverkusen, Bayerwerk
Bi/mö/Ma
IVa

## Verfahren zur Herstellung von 1-(2-Oxyaminosulfonyl-phenyl-sulfonyl)-3-heteroaryl-harnstoffen

Die Erfindung betrifft ein neues Verfahren zur Herstellung von 1-(2-Oxyaminosulfonyl-phenyl-sulfonyl)-3-heteroaryl-harnstoffen.

Es ist bekannt, daß man bestimmte 1-(2-Oxyaminosulfonyl-phenylsulfonyl)-3-heteroaryl-harnstoffe, wie z. B. 1-(2-[N-Methoxy-N-methyl-aminosulfonyl]-phenylsulfonyl)-3-(4,6-dimethoxy-pyrimidin-2-yl)-harnstoff, erhält, wenn man 2-Oxyaminosulfonyl-phenylsulfonyl-isocyanate, wie z. B. 2-(N-Methoxy-N-methyl-aminosulfonyl)-phenylsulfonyl-isocyanat, mit Heteroarylaminen, wie z. B. 2-Amino-4,6-dimethoxy-pyrimidin, umsetzt (vergl. US-PS 4 310 346). Die Synthese von 1-(2-Oxyaminosulfonyl-phenylsulfonyl)-3-heteroaryl-harnstoffen mit einer NH-Gruppierung im Oxyaminosulfonyl-Rest ist jedoch nach der genannten Methode bisher nicht gelungen.

Es wurde nun ein neues Verfahren zur Herstellung von 1-(2-Oxyaminosulfonyl-phenylsulfonyl)-3-heteroaryl-harnstoffen der allgemeinen Formel (I)

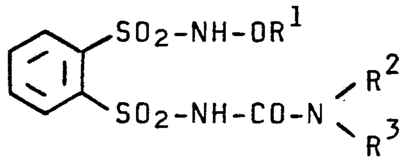

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für einen gegebenenfalls substituierten und/oder gegebenenfalls anellierten sechsgliedrigen aromatischen Heterocyclus, welcher wenigstens ein Stickstoffatom enthält, steht,

gefunden,

welches dadurch gekennzeichnet ist, daß man Benzodisultame der Formel (II)

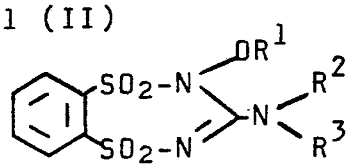

(II)

in welcher

$R^1$, $R^2$ und $R^3$ die oben angegebenen Bedeutungen haben,

mit Wasser gegebenenfalls in Gegenwart von Basen und gegebenenfalls in Gegenwart von Verdünnungsmitteln bei Temperaturen zwischen 0 °C und 100 °C umsetzt.

Es ist als überraschend anzusehen, daß die 1-(2-Oxyaminosulfonyl-phenylsulfonyl)-3-heteroaryl-harnstoffe der Formel (I) nach dem erfindungsgemäßen Verfahren durch selektive Ringöffnung von Benzodisultamen der Formel (II) hergestellt werden können, da neben dieser neuartigen Reaktion auch andere Spaltungsreaktionen, beispielsweise durch Angriff an den Sulfonyl-Gruppierungen zu erwarten waren.

Die beim erfindungsgemäßen Verfahren ablaufende chemische Reaktion kann beispielsweise durch folgendes Formelschema skizziert werden:

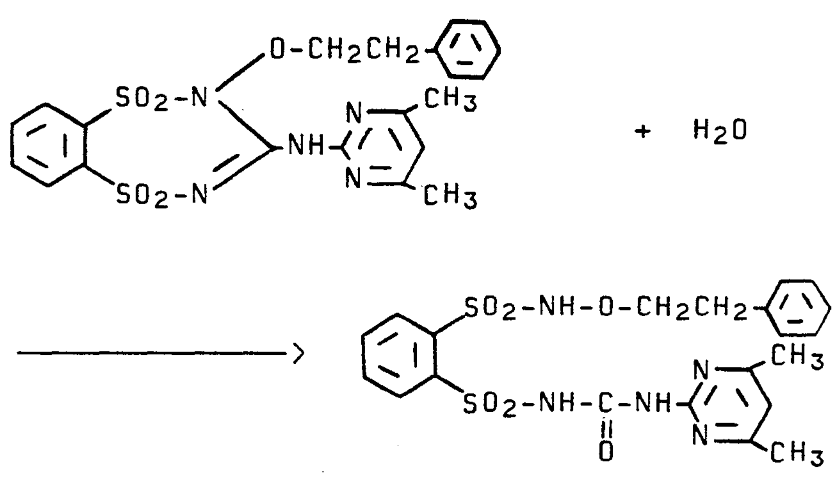

Die als Ausgangsstoffe zu verwendenden Benzodisultame sind durch die Formel (II) definiert.

Vorzugsweise stehen darin

$R^1$ für $C_1$-$C_{12}$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [ welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist],

$R^2$ für Wasserstoff, $C_1$-$C_4$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl , $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl, $C_3$-$C_6$-Alkinyl oder Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] und

$R^3$ für den Rest 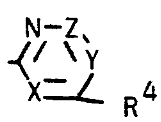 , worin

$R^4$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht,

X für Stickstoff oder eine Methin-Brücke (CH) steht,

Y für Stickstoff oder eine gegebenenfalls substituierte Methin-Brücke C-$R^5$ steht, wobei

$R^5$ für Wasserstoff, Fluor, Chlor, Brom oder $C_1$-$C_4$-Alkyl steht, und

Z für Stickstoff oder eine gegebenenfalls substituierte Methin-Brücke C-$R^6$ steht, wobei

$R^6$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], Amino, $C_1$-$C_4$-Alkylamino oder Di-($C_1$-$C_4$-alkyl)-amino steht.

Insbesondere bevorzugt sind Ausgangsstoffe der Formel (II), in welcher

$R^1$ für $C_1$-$C_8$-Alkyl [welches gegebenenfalls durch Fluor oder Chlor substituiert ist], $C_3$-$C_4$-Alkenyl, $C_1$-$C_2$-Alkoxy-carbonylmethyl, Phenylethyl oder Benzyl [welches gegebenenfalls durch Fluor, Chlor, Nitro, Cyano, Methyl, Methoxy oder Methoxy-carbonyl substituiert ist] steht,

$R^2$ für Wasserstoff steht und

$R^3$ für den Rest 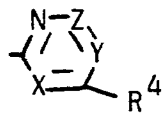 steht, worin

$R^4$ für Chlor, Methyl, Methoxy oder Ethoxy steht,

X für Stickstoff steht,

Y für eine Methin-Brücke (CH) steht, und

Z für eine gegebenenfalls substituierte Methin-Brücke C-$R^6$ steht, wobei

$R^6$ für Wasserstoff, Chlor, Methyl, Methoxy, Ethoxy, Methylthio, Ethylthio, Dimethylamino oder Diethylamino steht.

Beispiele für Ausgangsstoffe der Formel (II) sind in der nachstehenden Tabelle 1 aufgeführt:

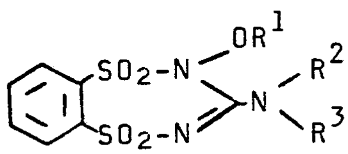

(II)

Tabelle 1: Beispiele für Ausgangsstoffe der Formel (II)

| $R^1$ | $R^2$ | $R^3$ |
|---|---|---|
| $-C_5H_{11}$ | H | 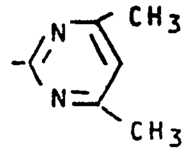 |
| $-C_8H_{17}$ | H | 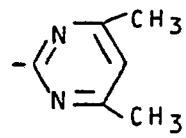 |
| 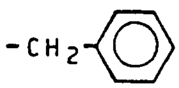 | H | 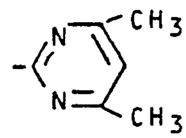 |
| $-CH_2-CH=CH_2$ | H | 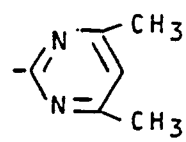 |
| 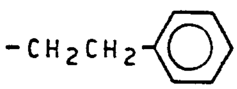 | H | 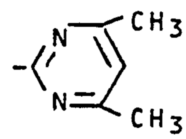 |
| 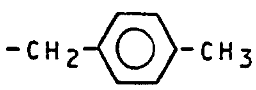 | H | 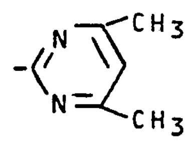 |
| 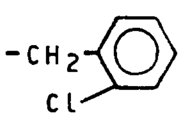 | H | 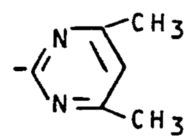 |
| 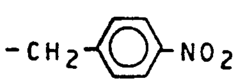 | H | 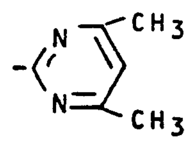 |
| $-CH(CH_3)-COOCH_3$ | H | 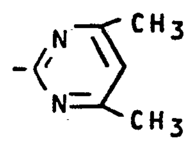 |

Die Verbindungen der Formel (II) sind bisher nicht in der Literatur beschrieben. Man erhält die Verbindungen der Formel (II), wenn man Benzol-1,2-disulfonsäure-dichlorid der Formel (III)

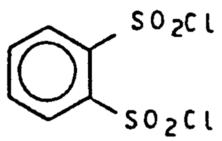

(III)

mit Oxyguanidin-Derivaten der Formel (IV)

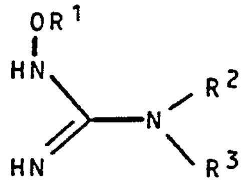

(IV)

in welcher

$R^1$, $R^2$ und $R^3$ die oben angegebene Bedeutung haben,

in Gegenwart von Säureakzeptoren, wie z.B. Pyridin oder Diazabicyclooctan (DABCO), und gegebenenfalls in Gegenwart von Verdünnungsmitteln, wie z.B. Methylenchlorid, Chloroform, Tetrahydrofuran oder Dioxan, bei Temperaturen zwischen -30°C und +50°C umsetzt.

Die Aufarbeitung kann nach üblichen Methoden erfolgen, beispielsweise durch Einengen, Aufnehmen des Rückstandes in Methylenchlorid, Waschen mit verdünnter Salzsäure und mit Wasser, Abtrennen, Trocknen, Filtrieren und Einengen der organischen Phase, wobei die Produkte der Formel (II) im Rückstand verbleiben.

Das als Ausgangsstoff zu verwendende Benzol-1,2-disulfonsäure-dichlorid der Formel (III) ist bereits bekannt (vgl. J.Org.Chem. 31, (1966), 3289-3292).

Die weiter als Ausgangsstoffe zu verwendenden Oxyguanidin-Derivate sind durch die Formel (IV) allgemein definiert. In Formel (IV) haben $R^1$, $R^2$ und $R^3$ vorzugsweise bzw. insbesondere die gleichen Bedeutungen, wie sie oben im Rahmen der Substituenten-definition der Formel (II) vorzugsweise bzw. als insbesondere bevorzugt angegeben sind.

Als Ausgangsstoffe der Formel (IV) seien beispielsweise genannt:

N'-(4-Methyl-pyrimidin-2-yl)-, N'-(4,6-Dimethyl-pyrimidin-2-yl)-,N'-(4-Methoxy-6-methyl-pyrimidin-2-yl)-,N'-(4-Ethoxy-6-methyl-pyrimidin-2-yl)-, N'-(4-Chlor-6-methoxy-pyrimidin-2-yl)-,N'-(4-Chlor-6-ethoxy-pyrimidin-2-yl)-,N'-(4-Chlor-6-dimethylamino-pyrimidin-2-yl)-,N'-(4-Methyl-6-methylthio-pyrimidin-2-yl)- und N'-(4-Dimethylamino-6-methyl-pyrimidin-2-yl)-N''-methoxy-guanidin, -N''-ethoxy-guanidin,-N''-propoxy-guanidin, -N''-isopropoxyguanidin, -N''-butoxy-guanidin, -N''-isobutoxy-guanidin, -N''-sec.-butoxy-guanidin, -N''-pentoxy-guanidin, -N''-isopentoxyguanidin, -N''-hexyloxy-guanidin, -N''-octyloxy-guanidin, -N''-allyloxy-guanidin, -N''-(2-chlor-ethoxy)-guanidin, -N''-(2-fluor-ethoxy)-guanidin, -N''-(2-chlor-propoxy)-guanidin, -N''-(2-fluor-propoxy)-guanidin, -N''-(3-chlor-propoxy)-guanidin, -N''-(4-chlor-butoxy)-guanidin, -N''-methoxycarbonylmethoxy-guanidin, -N''-ethoxycarbonylmethoxy-guanidin, -N''-(1-methoxycarbonyl-ethoxy)-guanidin, -N''-(1-ethoxycarbonylethoxy)-guanidin, -N''-dimethylaminocarbonylmethoxy-guanidin, -N''-(2-phenyl-ethoxy)-guanidin, -N''-phenoxy-guanidin, -N''-(4-methyl-benzyloxy)-guanidin, -N''-(4-fluor-benzyloxy)-guanidin, -N''-(4-chlor-benzyloxy)-guanidin, -N''-(4-nitrobenzyloxy)-guanidin, -N''-(2,6-dichlor-benzyloxy)-guanidin, -N''-(4-methoxycarbonyl-benzyloxy)-guanidin und -N''-(4-ethoxycarbonyl-benzyloxy)-guanidin.

Die Ausgangsstoffe der Formel (IV) sind zum Teil bekannt (vgl. J.Chem. Soc. 1962, 3915); zum Teil sind sie Gegenstand einer nicht zum vorveröffentlichten Stand der Technik gehörenden Patentanmeldung der Anmelderin (vgl. DE-OS 3 334 455).

Man erhält die Verbindungen der Formel (IV) wenn man Cyanamid-Derivate der Formel (V)

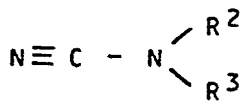

(V)

in welcher

$R^2$ und $R^3$ die oben angegebenen Bedeutungen haben,

mit Hydroxylamin-Derivaten der Formel (VI)

$$H_2N-OR^1 \qquad (VI)$$

in welcher

$R^1$ die oben angegebene Bedeutung hat,

bzw. mit Hydrochloriden von Hydroxylamin-Derivaten der Formel (VI)

gegebenenfalls in Gegenwart von Verdünnungsmitteln, wie z. B. Ethanol, Propanol oder Butanol, bei Temperaturen zwischen 20 °C und 120 °C umsetzt und gegebenenfalls die Umsetzungsprodukte mit Säureakzeptoren, wie z. B. Ammoniak, Kaliumcarbonat oder Natriumhydroxid, behandelt.

Die Cyanamid-Derivate der Formel ( V) sind zum Teil bekannt (vergl. J. Chem. Soc. 1953, 1725). Man erhält die Verbindungen der Formel ( V) im wesentlichen nach folgenden Synthesewegen:

(a) durch Umsetzung von Alkalimetall- oder Erdalkalimetall-Salzen von Cyanamid - wie z. B. Natriumcyanamid oder Calciumcyanamid - mit Chlor-hetarenen der Formel (VII)

$$Cl-R^3 \qquad (VII)$$

in welcher

$R^3$ die oben angegebene Bedeutung hat,

und gegebenenfalls anschließend - wenn $R^2$ nicht für Wasserstoff steht - mit Halogenverbindungen der Formel (VIII)

$$Q-R^2 \qquad (VIII)$$

in welcher

$R^2$ für einen gegebenenfalls substituierten Rest aus der Reihe Alkyl, Alkenyl, Alkinyl und Aralkyl steht, und

Q für Chlor, Brom oder Jod steht,

gegebenenfalls in Gegenwart von inerten Verdünnungsmitteln, wie z. B. Aceton, Acetonitril oder Dimethylformamid, bei Temperaturen zwischen 0 °C und 100 °C.

Nach Einengen und Auflösen des Rückstandes in Wasser können die Cyanamid-Derivate der Formel ( V) durch Ansäuern, z. B. mit Salzsäure, ausgefällt und durch Absaugen isoliert werden.

Alternativ erhält man die Verbindungen der Formel (V)

(b) für den Fall, daß $R^3$ für einen substituierten Pyrimidinylrest steht, durch Umsetzung von Cyanoguanidin ('Dicyandiamid') mit ß-Dicarbonylverbindungen, wie Acetylaceton (vergl. J. Chem. Soc. 1953, 1725 - 1730), Acetessigsäureestern (vergl. J. Prakt. Chem. 77 (1908), 542 und J. Chem.Soc. 1948, 586) oder Malonsäureestern (vergl. DE-PS 158 591).

Die aus Acetessigsäureestern bzw. Malonsäureestern erhaltenen 2-Cyanamino-4-hydroxy-6-methyl- bzw. -4,6-dihydroxy-pyrimidine können auf bekannte Weise durch Umsetzung mit Alkylierungsmitteln, wie z. B. Dimethyl- oder Diethylsulfat, gegebenenfalls in Gegenwart von Verdünnungsmitteln, wie z. B. Wasser, Methanol, Ethanol, n- und iso-Propanol, Aceton, Dioxan oder Dimethylformamid, und in Gegenwart von Säurebindemitteln, wie z. B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z. B. Acetanhydrid oder Acetylchlorid acyliert und nach der Alkylierung mit wässrigen Säuren oder Basen wieder entacyliert.

In einem weiteren Alternativverfahren erhält man die Verbindungen der Formel ( V), wenn man

(c) Amino-hetarene der Formel (IX)

$$H_2N-R^3 \qquad (IX)$$

in welcher

$R^3$ die oben angegebene Bedeutung hat,

mit Carbonylisothiocyanaten der Formel (X)

$$R^7-\overset{O}{\overset{\|}{C}}-N=C=S \qquad (X)$$

in welcher

$R^7$ für Ethoxy oder Phenyl steht,

gegebenenfalls in Gegenwart eines inerten Verdünnungsmittels, wie z. B. Aceton, Acetonitril oder Toluol, bei Temperaturen zwischen 0 °C und 100 °C umsetzt, die hierbei gebildeten Carbonylthioharnstoffe der Formel (XI)

$$R^7-\overset{O}{\overset{\|}{C}}-NH-\overset{S}{\overset{\|}{C}}-NH-R^3 \qquad (XI)$$

in welcher

$R^3$ und $R^7$ die oben angegebenen Bedeutung haben,

gegebenenfalls nach Einengen durch Absaugen isoliert und mit wässrigen Alkalimetall- oder Erdalkalimetallhydroxidlösungen, wie z. B. Natronlauge, gegebenen-

falls in Gegenwart eines organischen Lösungsmittels, wie z. B. Tetrahydrofuran oder Dioxan, bei Temperaturen zwischen 0 °C und 120 °C umsetzt und die nach Ansäuern, z. B. mit Salzsäure, kristallin erhaltenen Thioharnstoffe der Formel (XII)

$$H_2N-\overset{\overset{S}{\|}}{C}-NH-R^3 \qquad (XII)$$

in welcher

$R^3$ die oben angegebene Bedeutung hat,

durch Absaugen isoliert und mit Metallverbindungen, welche Schwefelwasserstoff binden können, wie z. B. mit Blei(II)-acetat, Kupfer(II)-acetat, Quecksilber-(II)-acetat oder Eisen(II)-acetat, in Gegenwart von wässrigen Alkalimetall- oder Erdalkalimetall-hydroxidlösungen, wie z. B. Natronlauge, bei Temperaturen zwischen 20 °C und 100 °C umsetzt, nach Ende der Umsetzung filtriert und das Filtrat mit einer Säure, wie z. B. Essigsäure, ansäuert. Die hierbei kristallin anfallenden Produkte der Formel ( V) können durch Absaugen isoliert werden.

Die Ausgangsstoffe für die vorausgehend unter (a), (b) und (c) beschriebenen Herstellungsverfahren für die Cyanamid-Derivate der Formel ( V) sind bekannt und/oder können nach an sich bekannten Verfahren hergestellt werden.

Hierzu gehören die Chlor-hetarene der Formel (VII) (vergl. J. Chem. Soc. (c) 1966, 2031; Chem. Pharm. Bull. 11

(1963), 1382 - 1388 und Arch. Pharm. 295 (1962), 649 - 657), die Halogenverbindungen der Formel (VIII) (handelsübliche Chemikalien), die Amino-hetarene der Formel (IX) (vergl. Chem. Pharm. Bull. 11, (1963), 1382 - 1388; J. Chem. Soc. 1946, 81 und US-PS 4 299 960) und die Carbonylisothiocyanate der Formel (X) (vergl. J. Heterocycl. Chem. 5 (1968), 837 und US-PS 4 160 037).

Das erfindungsgemäße Verfahren wird vorzugsweise in Wasser als Lösungsmittel durchgeführt. Als weitere Verdünnungsmittel kommen alle inerten organischen Lösungsmittel, vorzugsweise jedoch aprotisch polare Solventien in Betracht. Hierzu gehören Ketone, wie z. B. Aceton und Methylethylketon, Nitrile, wie z. B. Acetonitril und Propionsäurenitril, Dimethylsulfoxid, Sulfolan, 1,2-Dimethoxyethan und Dioxan.

Das erfindungsgemäße Verfahren wird vorzugsweise in Gegenwart von Basen durchgeführt. Als solche kommen vorzugsweise Alkalimetall- oder Erdalkalimetall-hydroxide, wie z. B. Natrium-, Kalium- und Calcium-hydroxid, tertiäre Amine, wie z. B. Pyridin oder Diazabicyclooctan (DABCO) in Betracht.

Die Reaktionstemperaturen können beim erfindungsgemäßen Verfahren innerhalb eines größeren Bereiches variiert werden. Im allgemeinen arbeit man zwischen 0 °C und + 100 °C, vorzugsweise zwischen 10 °C und +80 °C. Das erfindungsgemäße Verfahren wird im allgemeinen bei Normaldruck durchgeführt.

Zur Durchführung des erfindungsgemäßen Verfahrens setzt man je Mol Benzodisultam der Formel (II) im allgemeinen zwischen 1 und 100 Mol, vorzugsweise zwischen 5 und 50 Mol Wasser und gegebenenfalls zwischen 1 und 10 Mol, vorzugsweise zwischen 1 und 5 Mol einer Base ein.

Die Reaktionskomponenten werden gewöhnlich bei Raumtemperatur zusammengegeben und das Reaktionsgemisch wird bis zum Reaktionsende gerührt.

Die Aufarbeitung kann auf übliche Weise durchgeführt werden; beispielsweise indem man - z. B. mit Salzsäure - ansäuert, auf etwa die Hälfte des Volumens einengt und das kristallin anfallende Produkt der Formel (I) durch Absaugen isoliert.

Die nach dem erfindungsgemäßen Verfahren herzustellenden 1-(2-Oxyaminosulfonyl-phenylsulfonyl)-3-heteroaryl-harnstoffe der Formel (I) können als Herbizide verwendet werden (vgl. JP-Patentanmeldung Sho 59-21839 vom 10.02.1984 und die hiermit gleichzeitig eingereichte DE-Patentanmeldung P 34 31 929.8 (Le A 23 311); eine besonders gute herbizide Wirkung zeigt die gemäß nachfolgendem Beispiel 1 herstellbare Verbindung).

Herstellungsbeispiele

Beispiel 1

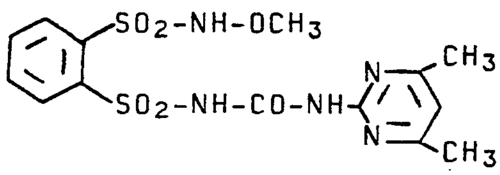

10,8 g (0,027 Mol) der Verbindung der nachstehenden Strukturformel

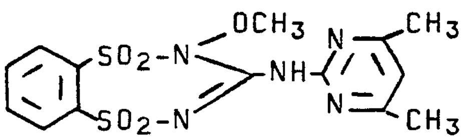

werden portionsweise zu einer Lösung von 4,0 g (0,1 Mol) Natriumhydroxid in 100 ml Wasser unter Rühren bei 20 °C gegeben. Das Reaktionsgemisch wird gerührt, bis eine klare Lösung entstanden ist und dann mit konzentrierter Salzsäure angesäuert. Das hierbei kristallin anfallende Produkt wird durch Absaugen isoliert.

Man erhält 9,5 g (90 % der Theorie) 1-(2-Methoxy-amino-sulfonyl-phenylsulfonyl)-3-(4,6-dimethyl-pyrimidin-2-yl)-harnstoff vom Schmelzpunkt 218 °C.

Zum gleichen Ergebnis kommt man, wenn man an Stelle von Wasser Mischungen von Wasser mit Acetonitril, Dioxan oder Tetrahydrofuran als Verdünnungsmittel verwendet.

Analog können die in der nachstehenden Tabelle 2 aufgeführten Verbindungen der Formel (I) hergestellt werden:

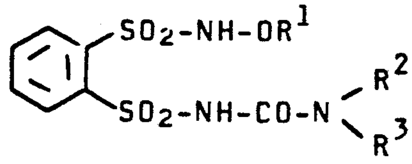

(I)

Tabelle 2

| Beisp.-Nr. | $R^1$ | $R^2$ | $R^3$ | Schmelzpunkt (°C) |
|---|---|---|---|---|
| 2 | $-C_4H_9$-n | H | 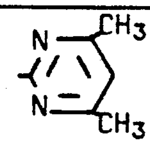 | 169 |
| 3 | $-C_3H_7$-n | H | 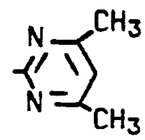 | 212 (zers.) |
| 4 | $-CH(CH_3)_2$ | H | 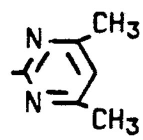 | 218 (zers.) |
| 5 | $-C_8H_{17}$-n | H | 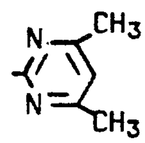 | 145 |
| 6 | $-CH_2$- 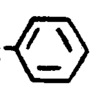 | H | 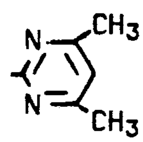 | 188 |

Tabelle 2 (Fortsetzung)

| Beisp.-Nr. | $R^1$ | $R^2$ | $R^3$ | Schmelzpunkt (°C) |
|---|---|---|---|---|
| 7 | $-CH_2-CH=CH_2$ | H | 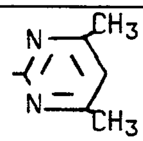 | 182 (Zers.) |
| 8 | $-C_2H_5$ | H | 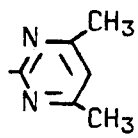 | 218 |
| 9 | $-CH_3$ | H | 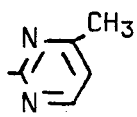 | 194-195 |
| 10 | $-CH_3$ | H | 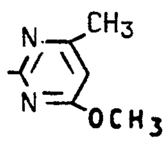 | 218 |

Herstellung der Ausgangsverbindungen der Formel (II)

Beispiel (II-1)

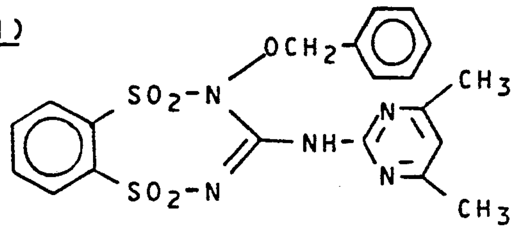

14g (0,05 Mol) Benzol-1,2-disulfonsäurechlorid werden portionsweise bei -20°C zu einer Mischung von 13,6 g (0,05 Mol) N'-(4,6-Dimethylpyrimidin-2-yl)-N''-benzyloxy-guanidin, 12 g (0,15 Mol) Pyridin und 100 ml Methylenchlorid gegeben. Man rührt 3 Stunden bei -20°C und 15 Stunden bei +20°C nach.

Dann wird das Reaktionsgemisch eingedampft und der Rückstand mit 70 ml Dioxan versetzt. Es wird filtriert. Das Filtrat wird eingeengt, mit Ethanol verrieben und das ausgefallene Produkt durch Absaugen isoliert.
Man erhält 15g (68 % der Theorie) der Verbindung der oben angegebenen Strukturformel vom Schmelzpunkt 199°C.

Analog können die in der nachstehenden Tabelle 3 aufgeführten Verbindungen der Formel (IIa) hergestellt werden:

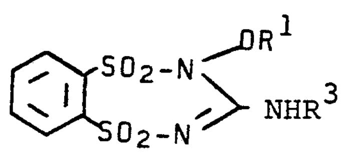

(IIa)

Tabelle 3:

| Beispiel Nr. | $R^1$ | $R^3$ | Schmelzpunkt (°C) |
|---|---|---|---|
| (II-2) | $-C_8H_{17}$ | 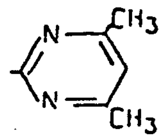 | 164 |
| (II-3) | $-CH_2CH_2-$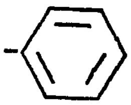 | 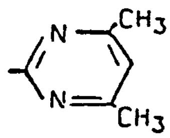 | |
| (II-4) | $-CH_3$ | 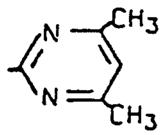 | 158 |
| (II-5) | $-C_2H_5$ | 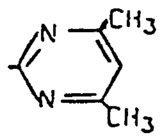 | 104 (Zers.) |
| (II-6) | $-C_3H_7$(-n) | 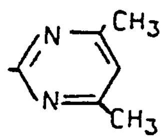 | 134 |
| (II-7) | $-C_3H_7$(-i) | 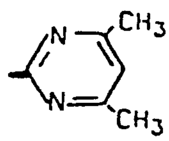 | amorph |
| (II-8) | $-C_4H_9$(-n) | 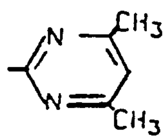 | 179 |

Tabelle 3 (Fortsetzung)

| Beispiel Nr. | $R^1$ | $R^3$ | Schmelzpunkt (°C) |
|---|---|---|---|
| (II-9) | $-CH_2-CH=CH_2$ | 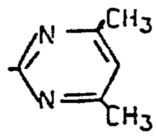 | 180 (Zers.) |
| (II-10) | $-CH_2-COOC_2H_5$ | 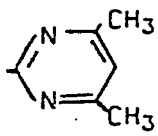 | 210 (Zers.) |
| (II-11) | $-CH_3$ | 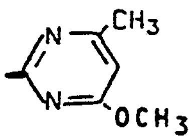 | 151 |
| (II-12) | $-CH_3$ | 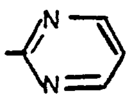 | 187 (Zers.) |

Herstellung der Ausgangsstoffe der Formel (IV):

Beispiel (IV-1)

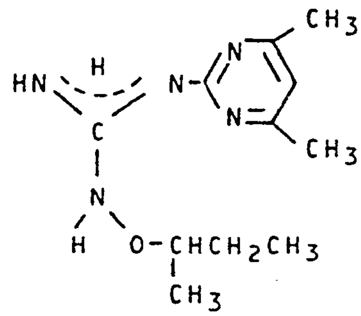

Eine Mischung aus 143g (0,97 Mol) 2-Cyanamino-4,6-dimethyl-pyrimidin, 94,3g (1,06 Mol) O-sec.-Butyl-hydroxylamin und 190 ml Ethanol wird 6 Stunden unter Rückfluß zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57 % der Theorie) N'-(4,6-Dimethyl-pyrimidin-2-yl)-N''-sec.-butoxy-guanidin vom Schmelzpunkt 52°C.

Analog können die in der nachstehenden Tabelle 4 aufgeführten Verbindungen der Formel (IV) hergestellt werden:

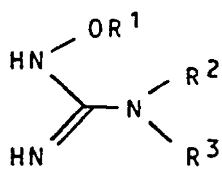

(IV)

Tabelle 4 :

| Beispiel Nr. | $R^1$ | $R^2$ | $R^3$ | Schmelzpunkt(°C) |
|---|---|---|---|---|
| (IV-2) | $-CH_2CH(CH_3)_2$ | H | 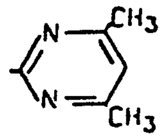 | 78 |
| (IV-3) | $-CH_2CH=CH_2$ | H | 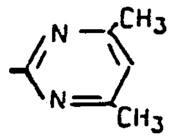 | 103 |
| (IV-4) | $-CH_2$ $CH_2$- 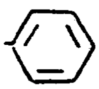 | H | 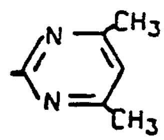 | $n_D^{24}$=1,5776 |
| (IV-5) | $-C_8H_{17}n$ | H | 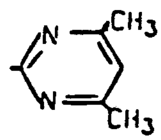 | 58 |
| (IV-6) | $-CH_2$- 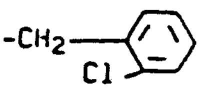 | H | 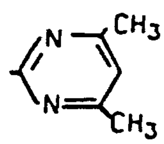 | 102-103 |
| (IV-7) | $-CH_2CH_2CH_2Cl$ | H | 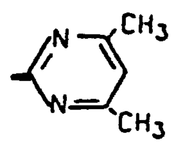 | 137 |
| (IV-8) | 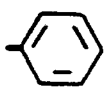 | H | 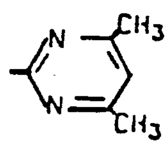 | 189-192 (Zers.) |

Tabelle 4 -Fortsetzung

| Beispiel Nr. | $R^1$ | $R^2$ | $R^3$ | Schmelzpunkt (°C) |
|---|---|---|---|---|
| (IV -9) | $-CH_2COOCH_3$ | H | 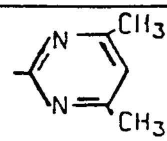 | 148-149 |
| (IV 10) | $-CH_2COOC_2H_5$ | H | 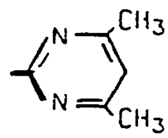 | 98- 99 |
| (IV 11) | $-\underset{CH_3}{\underset{\mid}{CH}}-COOCH_3$ | H | 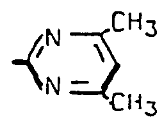 | 147-148 |
| (IV 12) | 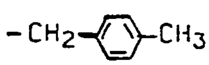 | H | 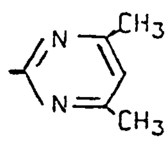 | 85- 86 |
| (IV 13) | 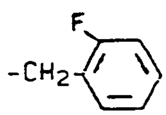 | H | 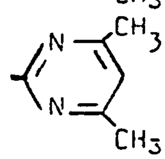 | 114-116 |
| (IV 14) | 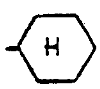 | H | 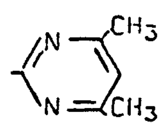 | |
| (IV -15) | 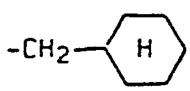 | H | 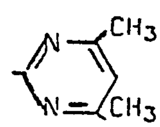 | |
| (IV -16) | $-CH_2CON(CH_3)_2$ | H | 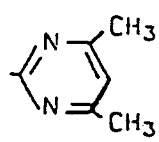 | |
| (IV -17) | $-CH_2OCH_3$ | H | 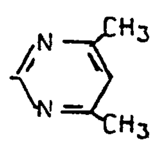 | |

Tabelle 4 -Fortsetzung

| Beispiel Nr. | $R^1$ | $R^2$ | $R^3$ | Schmelzpunkt (°C) |
|---|---|---|---|---|
| (IV-18) | $-CH_2SCH_3$ | H | 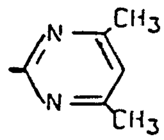 | |
| (IV-19) | 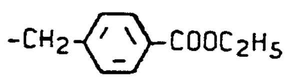 | H | 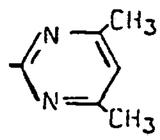 | 138 |
| (IV-20) | $-CH_2CF_3$ | H | 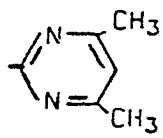 | |
| (IV-21) | 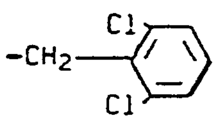 | H | 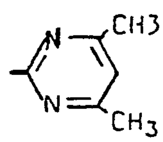 | 140-145 |
| (IV-22) | 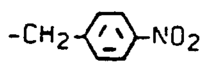 | H | 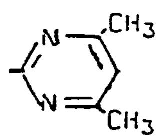 | 170-2 |
| (IV-23) | 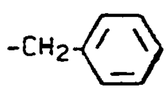 | H | 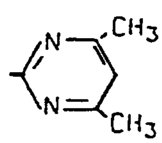 | 102 |
| (IV-24) | $CH_3$ | $CH_3$ | 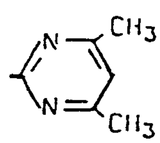 | 95 |

Tabelle 4 - Fortsetzung

| Beispiel Nr. | $R^1$ | $R^2$ | $R^3$ | Schmelzpunkt(°C) |
|---|---|---|---|---|
| (IV- 25) | $CH_3$ | $CH_3$ | 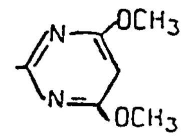 | 135 |
| (IV- 26) | $CH_3$ | H | 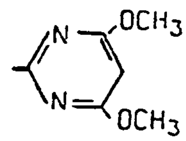 | 122 |
| (IV - 27) | $CH_3$ | H | 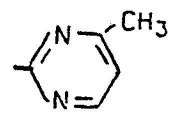 | 152 |
| (IV- 28) | $CH_3$ | H | 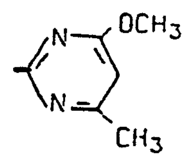 | 126 |
| (IV- 29) | $CH_3$ | H | 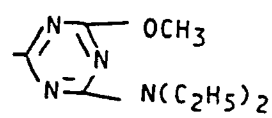 | 112 |
| (IV- 30) | $CH_3$ | H | 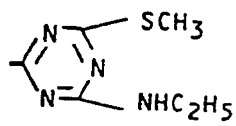 | 117 |

Tabelle 4 (Fortsetzung)

| Beispiel Nr. | $R^1$ | $R^2$ | $R^3$ | Schmelzpunkt (°C) |
|---|---|---|---|---|
| (IV-31) | $-CH(CH_3)_2$ | H | 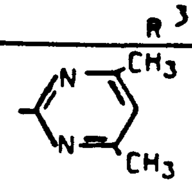 | 84 |
| (IV-32) | $-C_4H_9(n)$ | H | 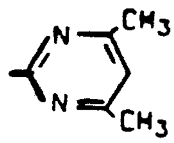 | Öl |
| (IV-33) | $-C_3H_7(-n)$ | H | 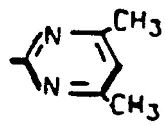 | Öl |
| (IV-34) | $-CH_2-COOC_3H_7(-i)$ | H | 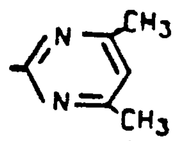 | 112 |
| (IV-35) | $-C_2H_5$ | H | 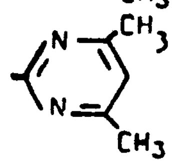 | 88 |
| (IV-36) | $-CH_3$ | H | 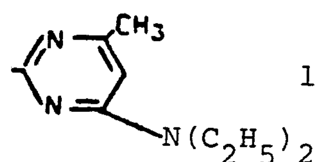 | 112 |
| (IV-37) | $-CH_2-CH(CH_3)_2$ | H | 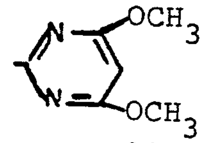 | 76 |
| (IV-38) | $-\underset{CH_3}{CH}-C_2H_5$ | H | 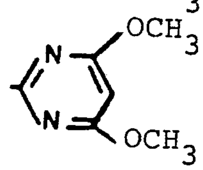 | 68 |
| (IV-39) | $-C_2H_5$ | H | 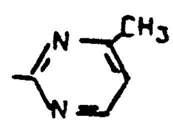 | 95 |

Tabelle 4 (Fortsetzung)

| Beispiel Nr. | $R^1$ | $R^2$ | $R^3$ | Schmelzpunkt (°C) |
|---|---|---|---|---|
| (IV-40) | $-CH_3$ | H | 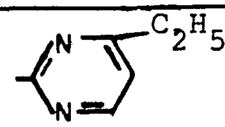 | 98 |
| (IV-41) | $-CH_3$ | H | 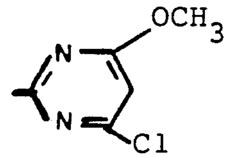 | 112 |
| (IV-42) | $-CH_3$ | H | 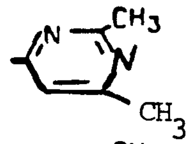 | 143 |
| (IV-43) | $-CH_3$ | H | 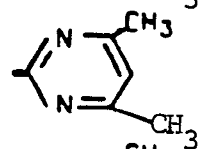 | 110 |
| (IV-44) | $-CH_2-COOC_2H_5$ | H | 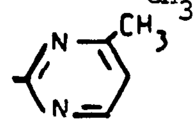 | |
| (IV-45) | $-CH_2-$ 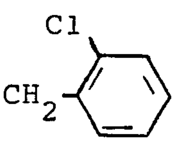 | H | 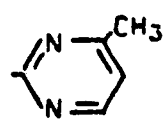 | 140 |
| (IV-46) | $-CH_2-$ 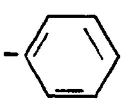 | H | 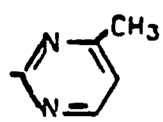 | 150 |
| (IV-47) | $-CH_2-$ 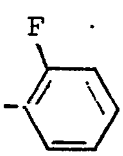 | H | 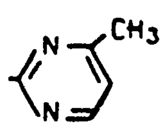 | 205 |
| (IV-48) | $-CH_2-CH=CH_2$ | H | 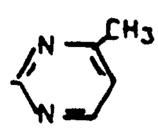 | |

Tabelle 4 (Fortsetzung)

| Beispiel Nr. | $R^1$ | $R^2$ | $R^3$ | Schmelzpunkt (°C) |
|---|---|---|---|---|
| (IV-49) | $-C_4H_9(-n)$ | H | 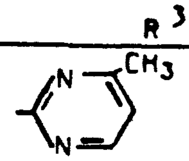 | |
| (IV-50) | $-C_4H_9(-s)$ | H | 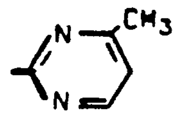 | |
| (IV-51) | $-CH_2CH_2CH_2-Cl$ | H | 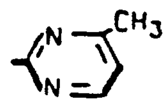 | ~ 102 |
| (IV-52) | $-CH_3$ | H | 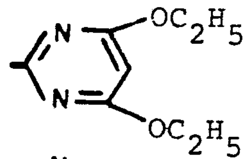 | |
| (IV-53) | $-CH_3$ | H | 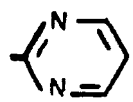 | 107-109 |
| (IV-54) | $-CH_2-$ 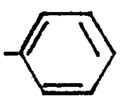 | H | 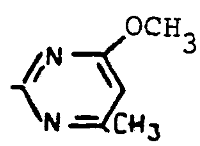 | $n_D^{20}$ = 1,5645 |
| (IV-55) | $-CH_2-$ 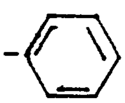 | H | 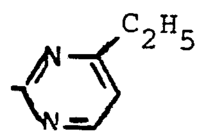 | 112 |
| (IV-56) | $-CH_2-$ 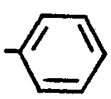 | H | 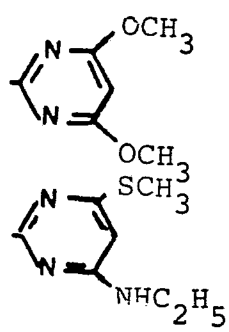 | 74 |
| (IV-57) | $-CH_2-$ 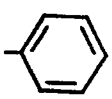 | H | | 122 |

Tabelle 4 (Fortsetzung)

| Beispiel Nr. | $R^1$ | $R^2$ | $R^3$ | Schmelzpunkt (°C) |
|---|---|---|---|---|
| (IV-58) | $-C_8H_7(-n)$ | H | 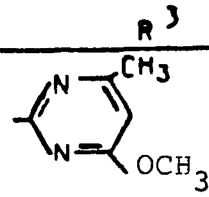 | 95 |
| (IV-59) | $-CH_2-$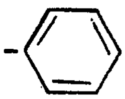 | H | 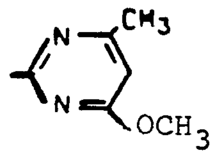 | 112 |
| (IV-60) | $-CH_2CH_2CH_2CH_2-Cl$ | H | 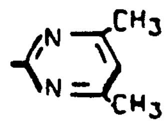 | Öl |
| (IV-61) | $-CH_2-CH_2-Cl$ | H | 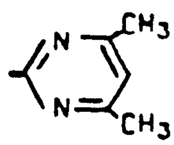 | Öl |
| (IV-62) | -CH(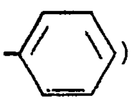$)_2$ | H | 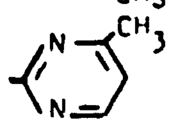 | 165 |

Herstellung der Ausgangsstoffe der Formel (V)

Beispiel (V-1)

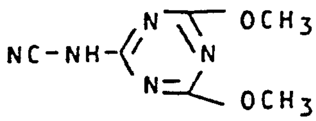

52,7 g (0,3 Mol) 2-Chlor-4,6-dimethoxy-s-triazin werden zu einer Lösung von 30 g (0,3 Mol) Cyanamid-Dinatriumsalz in 600 ml Aceton gegeben, und das Reaktionsgemisch wird 6 Stunden unter Rückfluß zum Sieden erhitzt. Nach Abdestillieren des Lösungsmittels wird der kristalline Rückstand in 250 ml Wasser gelöst und die Lösung mit konzentrierter Salzsäure angesäuert. Das kristallin angefallene Produkt wird durch Absaugen isoliert.

Man erhält 33 g (61 % der Theorie) 2-Cyanamino-4,6-dimethoxy -s-triazin mit einem Schmelzpunkt über 300°C.

Beispiel (V-2)

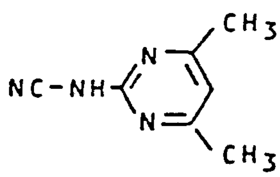

Ein Gemisch aus 42 g (0,5 Mol) Cyanoguanidin ('Dicyandiamid') und 50 g (0,5 Mol) 2,4-Pentandion ('Acetylaceton') wird 15 Stunden auf 120°C erhitzt. Dann wird nach Abkühlen das Reaktionsgemisch mit 500 ml Wasser versetzt und die Lösung bei 0°C bis 10°C mit Salzsäure angesäuert. Das hierbei kristallin angefallene Produkt wird durch Absaugen isoliert. Man erhält 51,8 g (70 % der Theorie) 2-Cyanamino-4,6-dimethyl-pyrimidin vom Schmelzpunkt 205°C.

Beispiel (V-3)

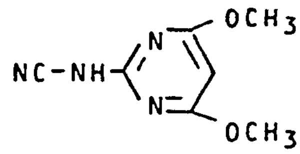

Eine auf 100°C erhitzte Lösung von 24 g (0,427 Mol) Kaliumhydroxid in 100 ml Wasser wird bei 100°C unter Rühren zu einer Mischung von 9,2g (0,043 Mol) 4,6-Dimethoxypyrimidin-2-yl-thioharnstoff und 70 ml Wasser gegeben. Man rührt 2 Minuten bei 100°C nach und gibt dann eine auf 100°C erwärmte Lösung von 16,2g (0,05 Mol) Blei-II-acetat in 30 ml Wasser hinzu. Man erhitzt noch 5 Minuten unter Rückfluß, kühlt dann auf 0°C bis 5°C ab und versetzt die wässrige Lösung mit 30ml Eisessig. Das hierbei kristallin ausfallende Produkt wird durch Absaugen isoliert.
Man erhält 6,3g (81,5 % der Theorie) 2-Cyanamino-4,6-dimethoxy-pyrimidin vom Schmelzpunkt 202°C.

Nach dem im vorausgehenden Beispiel exemplarisch beschriebenen Verfahren können die in der nachstehenden Tabelle 5 aufgeführten Verbindungen der Formel (V) hergestellt werden:

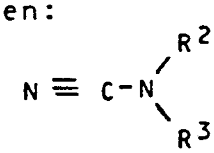

(V)

Tabelle 5

| Bsp. Nr. | $R^2$ | $R^3$ | Schmelzpunkt(°C) |
|---|---|---|---|
| (V-4) | H | 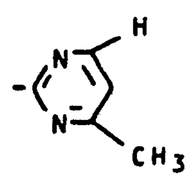 | 203(Zer.) |

Tabelle 5-Fortsetzung:

| Bsp. Nr. | $R^2$ | $R^3$ | Schmelzpunkt (°C) |
|---|---|---|---|
| (V-5) | H | 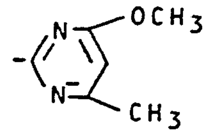 | 258 |
| (V-6) | H | 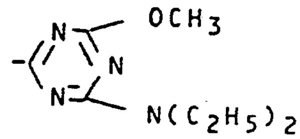 | 114 |
| (V-7) | H | 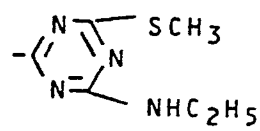 | |
| (V-8) | H | 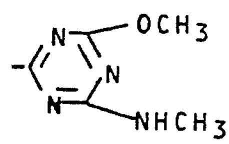 | 210 |
| (V-9) | H | 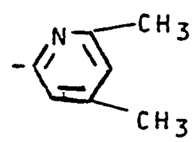 | |
| (V-10) | H | 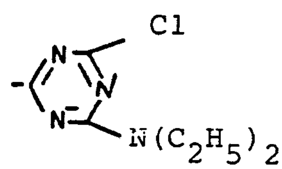 | 156 |
| (V-11) | H | 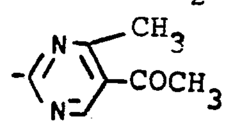 | 174 |
| (V-12) | H | 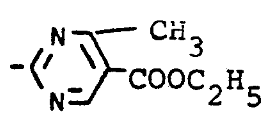 | 126 |
| (V-13) | H | 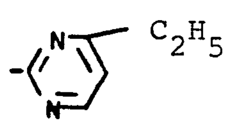 | 146 |

Tabelle 5-Fortsetzung:

| Bsp. Nr. | $R^2$ | $R^3$ | Schmelzpunkt(°C) |
|---|---|---|---|
| (V-14 ) | H | 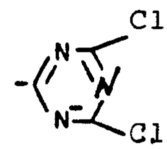 | > 250 |
| (V- 15) | H | 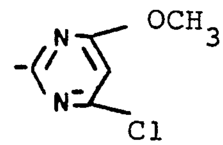 | 200 |
| (V- 16) | H | 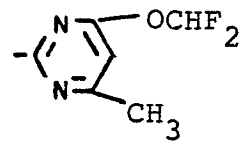 | 174 |
| (V- 17) | H | 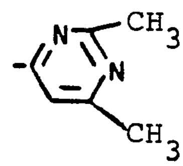 | 234 |
| (V- 18) | H | 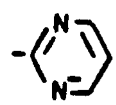 | 186 |
| (V-19) | H | 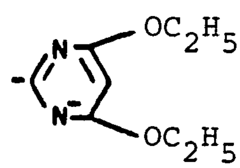 | |
| (V-20) | H | 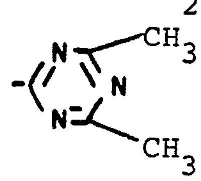 | 232 |

2-(Alkyl-cyano-amino)-pyrimidine der Formel (V) können beispielsweise wie folgt hergestellt werden:

Beispiel (V-21)

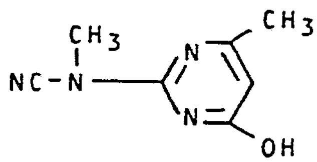

12,6g (0,1 Mol) Dimethylsulfat werden zu einer Lösung von 15 g (0,1 Mol) 2-Cyanamino-4-hydroxy-6-methyl-pyrimidin und 4,1g (0,1 Mol) Natriumhydroxid in 60 ml Wasser tropfenweise gegeben, wobei die Reaktionstemperatur von 20°C auf 40°C steigt. Nach zweistündigem Rühren bei 20°C wird das kristallin angefallene Produkt durch Absaugen isoliert.
Man erhält 11,1g (68 % der Theorie) 2-(Methyl-cyan-amino)-4-hydroxy-6-methyl-pyrimidin vom Schmelzpunkt 290°C.

Analog erhält man:
Beispiel (V-22) :

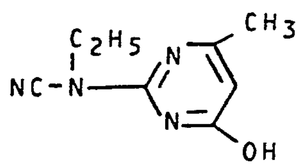

Fp: 215°C bis 220°C.

Beispiel (V-23)

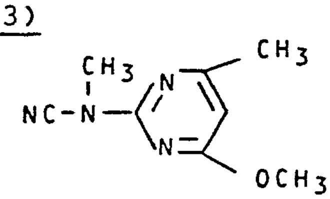

127,5 g (1 Mol) Dimethylsulfat werden zu einer Lösung von 75 g (0,5 Mol) 2-Cyanamino-4-hydroxy-6-methyl-pyrimidin -hergestellt nach Verfahren (b)- und 44 g (1,1 Mol) Natriumhydroxid in 750 ml Wasser tropfenweise gegeben,

wobei die Reaktionstemperatur von 20°C auf 35°C ansteigt. Nach zwölfstündigem Rühren bei 20°C wird durch Zugabe von Natronlauge ein pH-Wert zwischen 9 und 10 eingestellt und das kristallin angefallene Produkt durch Absaugen isoliert.

Man erhält 13 g (15 % der Theorie) 2-(Methyl-cyano-amino)-4-methoxy-6-methyl-pyrimidin vom Schmelzpunkt 123°C.

Analog erhält man

Beispiel (V-24):

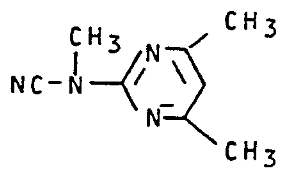

Fp: 104°C.

Beispiel (V-25)

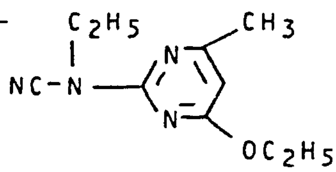

Fp: 71°C.

Herstellung der Ausgangsstoffe der Formel (XI)

Beispiel (XI-1)

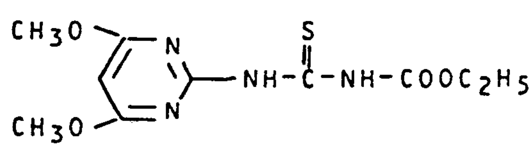

Eine Mischung aus 15,5g (0,1 Mol) 2-Amino-4,6-dimethoxy-pyrimidin, 13,1 g (0,1 Mol) Ethoxycarbonylisothiocyanat und 200 ml Acetonitril wird 2 Stunden bei 60°C gerührt. Dann wird auf 10°C abgekühlt, und das kristallin angefallene Produkt durch Absaugen isoliert.

Man erhält 22,5 g (79% der Theorie) 1-(Ethoxycarbonyl)-3-(4,6-dimethoxy-pyrimidin-2-yl)-thioharnstoff vom Schmelzpunkt 194°C (Zers.).

Nach dem im vorausgehenden Beispiel exemplarisch beschriebenen Verfahren können die in der nachstehenden Tabelle 6 aufgeführten Verbindungen der Formel (XI ) hergestellt werden:

$$R^7-\overset{O}{\overset{\|}{C}}-NH-\overset{S}{\overset{\|}{C}}-NH-R^3 \qquad (XI)$$

Tabelle 6

| Beispiel Nr. | $R^7$ | $R^3$ | Schmelzpunkt(°C) |
|---|---|---|---|
| (XI -2) | 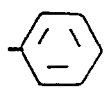 | 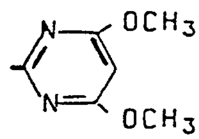 | 189 |
| (XI-3 ) | 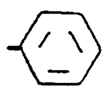 | 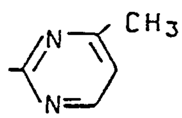 | 198-9 (Zers.) |
| (XI-4 ) | $-OC_2H_5$ | 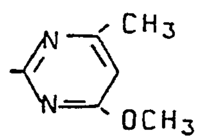 | 217 |
| (XI -5) | 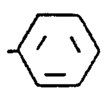 | 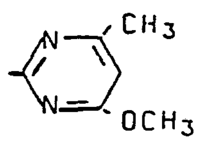 | 190 |
| (XI -6) | $-OC_2H_5$ | 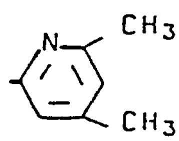 | 140 |
| (XI -7 ) | 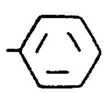 | 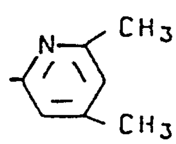 | 145 |

Tabelle 6 (Fortsetzung)

| Bsp. Nr. | $R^7$ | $R^3$ | Schmelzpunkt(°C) |
|---|---|---|---|
| XI-8 | 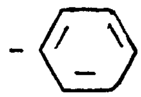 | 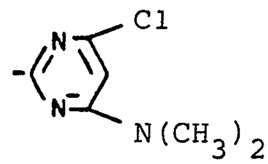 | 168 |
| XI-9 | 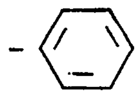 | 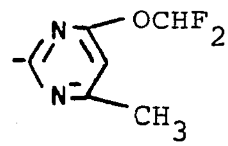 | 182 |
| XI-10 | $-OC_2H_5$ | 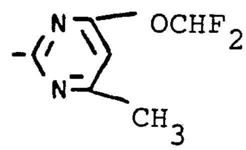 | 184-185 |
| XI-11 | $-OC_2H_5$ | 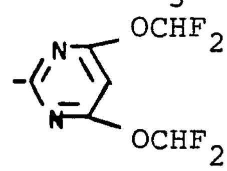 | 173 |
| XI-12 | $-OC_2H_5$ | 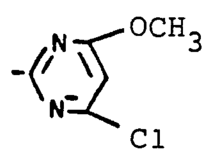 | 100 |
| XI-13 | 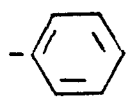 | 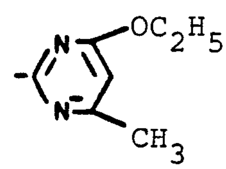 | 156 |
| XI-14 | 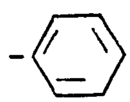 | 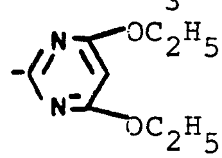 | 179 |
| XI-15 | $-OC_2H_5$ | 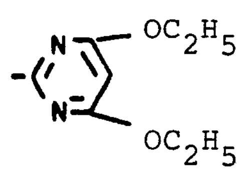 | 159 |
| XI-16 | 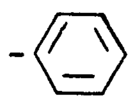 | 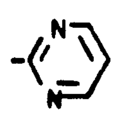 | 172-173 |

Herstellung der Ausgangsstoffe der Formel (XII)

Beispiel (XII-1)

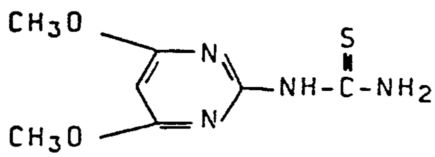

Eine Mischung von 5,0 g (0,0175 Mol) 1-(Ethoxycarbonyl)-3-(4,6-dimethoxy-pyrimidin-2-yl)-thioharnstoff, von 4,0 g (0,1 Mol) Natriumhydroxid und von 100 ml Wasser wird 2 Tage bei 20°C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94% der Theorie) 4,6-Dimethoxypyrimidin-2-yl-thioharnstoff vom Schmelzpunkt 245-8°C(Zers.).

Nach dem im vorausgehenden Beispiel exemplarisch beschriebenen Verfahren können die in der nachstehenden Tabelle 7 aufgeführten Verbindungen der Formel (XII) hergestellt werden:

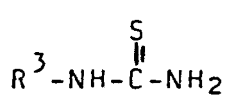

(XII)

Tabelle 7:

| Beispiel Nr. | $R^3$ | Schmelzpunkt (°C) |
|---|---|---|
| (XII-2) | 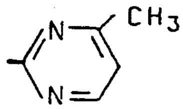 | 264-265 (Zers.) |
| (XII-3) | 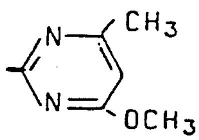 | 205-207 (Zers.) |
| (XII-4) | 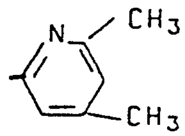 | 259-260 (Zers.) |

Tabelle 7 (Fortsetzung)

| Bsp. Nr. | $R^3$ | Schmelzpunkt (°C) |
|---|---|---|
| XII-5 | 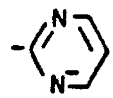 | 263 |
| XII-6 | 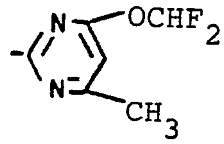 | 192-194 |
| XII-7 | 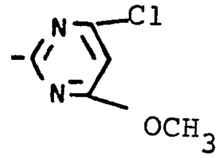 | 225-227 |
| XII-8 | 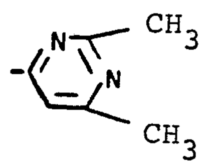 | 248 |
| XII-9 | 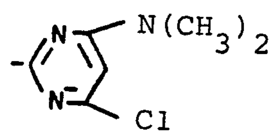 | |
| XII-10 | 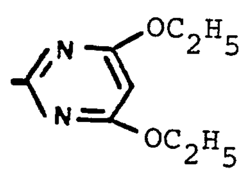 | 166 |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die gemäß Beispiel 1 herzustellende Verbindung zeigt in diesem Test eine sehr gute herbizide Wirkung.

Patentansprüche

1) Verfahren zur Herstellung von 1-(2-Oxyaminosulfonyl-phenyl-harnstoffen der allgemeinen Formel (I)

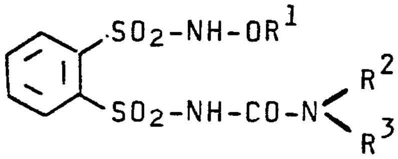

(I)

in welcher

$R^1$ für einen gegebenenfalls substituierten Rest aus der Reihe Alkyl, Alkenyl, Alkinyl, Cycloalkyl, Cycloalkylalkyl, Aralkyl und Aryl steht,

$R^2$ für Wasserstoff oder für einen gegebenenfalls substituierten Rest aus der Reihe Alkyl, Alkenyl, Alkinyl und Aralkyl steht und

$R^3$ für einen gegebenenfalls substituierten und/oder gegebenenfalls anellierten sechsgliedrigen aromatischen Heterocyclus, welcher wenigstens ein Stickstoffatom enthält, steht,

dadurch gekennzeichnet, daß man Benzodisultame der Formel (II)

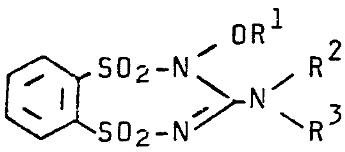

(II)

in welcher

$R^1$, $R^2$ und $R^3$ die oben angegebenen Bedeutungen haben,

mit Wasser gegebenenfalls in Gegenwart von Basen und gegebenenfalls in Gegenwart von Verdünnungsmitteln bei Temperaturen zwischen 0 °C und 100 °C umsetzt.

Europäisches Patentamt

# EUROPÄISCHER RECHERCHENBERICHT

**0 173 324**

EP 85110838.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| A | EP - A1 - 0 116 518 (CIBA)<br>* Formel I; Seiten 8,9 *<br>-- | 1 | C 07 D 239/42<br>C 07 D 239/47<br>C 07 D 239/48<br>C 07 D 239/54<br>C 07 D 251/16<br>C 07 D 251/18<br>C 07 D 251/22<br>C 07 D 251/46<br>C 07 D 251/50<br>C 07 D 213/75<br>A 01 N 47/36 |
| A | EP - A1 - 0 074 282 (DU PONT)<br>* Ansprüche 1,24 *<br>-- | 1 | |
| A | EP - A1 - 0 048 143 (DU PONT)<br>* Ansprüche 1,22 *<br>-- | 1 | |
| P,A | EP - A1 - 0 128 274 (SCHERING)<br>* Formel I; Anspruch 13 *<br>-- | 1 | |
| A | EP - A2 - 0 102 925 (CIBA)<br>* Formel I; Ansprüche 14-17 *<br>-- | 1 | RECHERCHIERTE SACHGEBIETE (Int. Cl 4) |
| A | EP - A3 - 0 085 028 (CIBA)<br>* Zusammenfassung *<br>---- | 1 | C 07 D 239/00<br>C 07 D 251/00<br>C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 17-10-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82